# EUROPÄISCHE PATENTSCHRIFT

(11) **EP 2 830 676 B1**
(45) Veröffentlichungstag und Bekanntmachung des Hinweises auf die Patenterteilung: **06.09.2017**
(21) Anmeldenummer: 13714552.0
(22) Anmeldetag: 27.03.2013
(51) Int. Cl.: A61M 1/16

(54) **HEIZVORRICHTUNG ZUM ERWÄRMEN VON DIALYSIERFLÜSSIGKEIT, DIALYSIERFLÜSSIGKEITS-SCHLAUCHSATZ, SET, MEDIZINISCHE VORRICHTUNG UND VERFAHREN**
HEATING DEVICE FOR HEATING DIALYSIS LIQUID, DIALYSIS LIQUID HOSE SET, SET, MEDICAL DEVICE AND METHOD
DISPOSITIF DE CHAUFFAGE POUR CHAUFFER UN LIQUIDE DE DIALYSE, JEU DE TUBULURES POUR LIQUIDE DE DIALYSE, TROUSSE, APPAREIL MÉDICAL ET PROCÉDÉ

(30) Priorität: 28.03.2012 DE 102012006149; 28.03.2012 US 201261616441 P
(43) Veröffentlichungstag der Anmeldung: 04.02.2015
(73) Patentinhaber: Fresenius Medical Care Deutschland GmbH, 61352 Bad Homburg (DE)
(72) Erfinder: BEISSER, Nicolas, 63454 Hanau (DE); OESTERREICH, Stefan, 61267 Neu-Anspach (DE)
(74) Vertreter: Bobbert & Partner Patentanwälte PartmbB
(86) Internationale Anmeldenummer: PCT/EP2013/000928
(87) Internationale Veröffentlichungsnummer: WO 2013/143694

(56) Entgegenhaltungen:
- WO-A2-2009/106354
- WO-A2-2010/025824
- DE-A1-102010 036 295
- US-A1- 2009 009 179
- US-A1- 2009 312 694

## Beschreibung

Die vorliegende Erfindung betrifft eine Heizvorrichtung gemäß Anspruch 1. Sie betrifft ferner ein Set gemäß Anspruch 9 sowie eine medizinische Vorrichtung gemäß Anspruch 10.

Aus der WO 2010/025824 A2 sind Vorrichtungen bekannt, mittels welcher Dialysierflüssigkeit während einer Dialysebehandlung mittels eines Heizbeutels erwärmt wird. Die Erwärmung erfolgt beim Durchströmen des Heizbeutels, bevor die Dialysierflüssigkeit in den Dialysator oder Blutbehandlungsfilter, in welchem ein Stoffaustausch über eine - üblicherweise semipermeable - Membran zwischen Blut und Dialysierflüssigkeit erfolgt, eingebracht wird.

Bei manchen Betriebsbedingungen ist ohne Gegenmaßnahmen getroffen zu haben allerdings nicht auszuschließen, dass der Heizbeutel aufgrund des von der Pumpe, welche die Dialysierflüssigkeit durch den Heizbeutel fördert, aufgebauten Drucks platzt. Ein Platzen könnte vor allem bei Anordnungen erfolgen, in welchen der Heizbeutel - beispielsweise zum Vermeiden von Ausfällungen im Schlauchsystem - auf der Druckseite der Pumpe angeordnet ist. Das Platzen des Heizbeutels kann eine Gefährdung für den Patienten bedeuten, jedenfalls dann, wenn dies nicht rechtzeitig bemerkt werden sollte.

Eine Aufgabe der vorliegenden Erfindung ist es, Vorrichtungen vorzuschlagen zum Senken der Gefahr, dass ein Heizbeutel während der Dialysebehandlung platzt oder unbemerkt platzt.

Die erfindungsgemäße Aufgabe wird durch eine Heizvorrichtung mit den Merkmalen des Anspruchs 1 gelöst.

So wird erfindungsgemäß eine Heizvorrichtung vorgeschlagen zum Erwärmen einer Dialysierflüssigkeit, welche in einem Heizbeutel vorliegt. Der Heizbeutel wird von der Dialysierflüssigkeit zu ihrem Erwärmen im Gebrauch durchströmt oder ist von ihr durchströmbar. Die Heizvorrichtung weist einen Aufnahmeabschnitt zum Aufnehmen des Heizbeutels auf, welcher derart ausgestaltet ist, dass er eine Verformung des Heizbeutels infolge eines Beutelinnendrucks in nur eine Erstreckungsrichtung des Heizbeutels zulässt. Zudem weist die Heizvorrichtung eine Sensorik auf. Die Sensorik ist angeordnet, vorgesehen, ausgestaltet, konfiguriert und/oder programmiert, um eine Verformung oder Längsdehnung des Heizbeutels oder die Lage oder Position des Heizbeutels, welche dieser innerhalb der Heizvorrichtung oder innerhalb des Aufnahmeabschnitts der Heizvorrichtung einnimmt, festzustellen oder zu überwachen.

Erfindungsgemäß wird ferner ein Set mit den Merkmalen des Anspruchs 9 vorgeschlagen. Das Set gemäß der vorliegenden Erfindung weist eine erfindungsgemäße Heizvorrichtung mit wenigstens einem Aufnahmeabschnitt für einen Heizbeutel auf. Ferner weist das Set wenigstens einen Dialysierflüssigkeits-Schlauchsatz auf, welcher vorgesehen oder ausgestaltet ist, um in die Heizvorrichtung eingelegt zu werden und zudem - vorzugsweise integral hiermit ausgestaltet - wenigstens einen von der Dialysierflüssigkeit durchströmbaren Heizbeutel aufweist.

Der Dialysierflüssigkeits-Schlauchsatz oder der Aufnahmeabschnitt des Sets - oder beide - sind derart ausgewählt oder festgelegt oder aufeinander abgestimmt, dass die Länge des Heizbeutels stets kleiner ist als die Länge des Aufnahmeabschnitts der Heizvorrichtung. Auf diese Weise ist sichergestellt, dass der Aufnahmeabschnitt den hierin eingelegten Heizbeutel bei dessen Gebrauch über dessen gesamte Länge radial begrenzt oder jedenfalls begrenzen könnte. Dies bedeutet, dass der Heizbeutel oder Abschnitte hiervon das Innere des Aufnahmeabschnitts selbst dann, wenn sich der Heizbeutel dehnt - und ggf. bis zum Platzen dehnt - nicht verlassen können.

Des Weiteren wird erfindungsgemäß eine medizinische Vorrichtung mit den Merkmalen des Anspruchs 10 vorgeschlagen, welche wenigstens eine erfindungsgemäße Heizvorrichtung aufweist oder hiermit verbunden ist.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll weitere erfindungsgemäße Ausführungsformen erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

Unter einer Erstreckungsrichtung wird in gewissen Ausführungsformen der vorliegenden Erfindung die Längsachse des Heizbeutels oder des Aufnahmeabschnitts verstanden.

Die Sensorik ist in gewissen Ausführungsformen der vorliegenden Erfindung eine rein mechanisch wirkende oder funktionierende Vorrichtung oder beides, welche keine elektronischen Bauteile aufweist. In anderen Ausführungsformen der vorliegenden Erfindung ist die Sensorik eine rein elektronisch wirkende oder funktionierende Vorrichtung oder beides. In einigen Ausführungsformen der vorliegenden Erfindung weist die Sensorik sowohl mechanisch als auch elektronisch oder optisch wirkende oder funktionierende, oder beides, Bauteile auf.

In bestimmten Ausführungsformen der vorliegenden Erfindung weist die Sensorik zur Überwachung der Verformung des Heizbeutels wenigstens einen mechanischen, einen optischen Sensor, ein verschiebbar gelagertes Element, einen Druckschalter, oder beliebige Kombinationen der vorstehend genannten Elemente auf oder besteht hieraus.

In manchen Ausführungsformen der vorliegenden Erfindung ist die Sensorik zur Erfassung der Längenausdehnung des Heizbeutels, vorzugsweise eines Stirnendes des Heizbeutels, insbesondere seines Kopfabschnittes, ausgestaltet oder angeordnet oder beides.

Die Sensorik ist in manchen Ausführungsformen der vorliegenden Erfindung keine Wiegeeinrichtung oder weist keine solche auf. Wiegeeinrichtungen können beispielsweise nach dem Prinzip arbeiten, dass ein Gewicht, etwa des Heizbeutels, oder dessen Gewichtsveränderung mittels Biegebalken und Dehnungsmessstreifen, welche die Durchbiegung des Biegebalkens oder eine andere Veränderung bestimmen, erfasst wird.

In bestimmten Ausführungsformen der vorliegenden Erfindung ist der Aufnahmeabschnitt derart ausgestaltet, dass er den Heizbeutel in einer Umfangsrichtung hiervon - oder in einer Ebene senkrecht zur Strömungsrichtung der Dialysierflüssigkeit innerhalb des Heizbeutels oder zur Längsachse des Heizbeutels - bei dessen Gebrauch umschließt. Dabei wird der Heizbeutel entlang seiner gesamten Länge umschlossen.

Ein "Umschließen" bedeutet in einigen Ausführungsformen der vorliegenden Erfindung, dass der Aufnahmeabschnitt den Heizbeutel während dessen Gebrauch entlang des gesamten Umfangs des Heizbeutels umgibt oder diesem anliegt. Alternativ umgibt der Aufnahmeabschnitt den Heizbeutel nicht entlang des gesamten Umfangs, sondern nur zu jeweils wenigstens 70 %, 80 %, 90 % oder 95 %, oder der Aufnahmeabschnitt umgibt den Heizbeutel in radialer Richtung doch wenigstens so sehr, dass der Heizbeutel nur entlang seiner Längsrichtung aus dem Aufnahmeabschnitt herausgezogen werden kann, nicht jedoch in seiner Querrichtung. Letzteres gilt für den Heizbeutel im befüllten Zustand oder im leeren Zustand oder unabhängig vom Füllungszustand des Heizbeutels.

In manchen Ausführungsformen der vorliegenden Erfindung ist die Heizvorrichtung ausgestaltet, um den durchströmbaren Heizbeutel in jedem Betriebzustand zylindrisch, manschettenartig oder rohrförmig zu umschließen oder dem Heizbeutel auf diese Weise anzuliegen. Dabei kann die o.g. Definition von "umschließen" gelten.

Der Aufnahmeabschnitt ist in einigen Ausführungsformen der vorliegenden Erfindung ausgestaltet, um einem Innendruck des Heizbeutels von 1050mbar über einen Zeitraum von 72 Stunden standzuhalten. Er muss in diesen Ausführungsformen ferner 10 Lastfällen über einen Zeitraum von jeweils 20 Sekunden mit einem Druck von jeweils 3200mbar standhalten und ist entsprechend ausgestaltet.

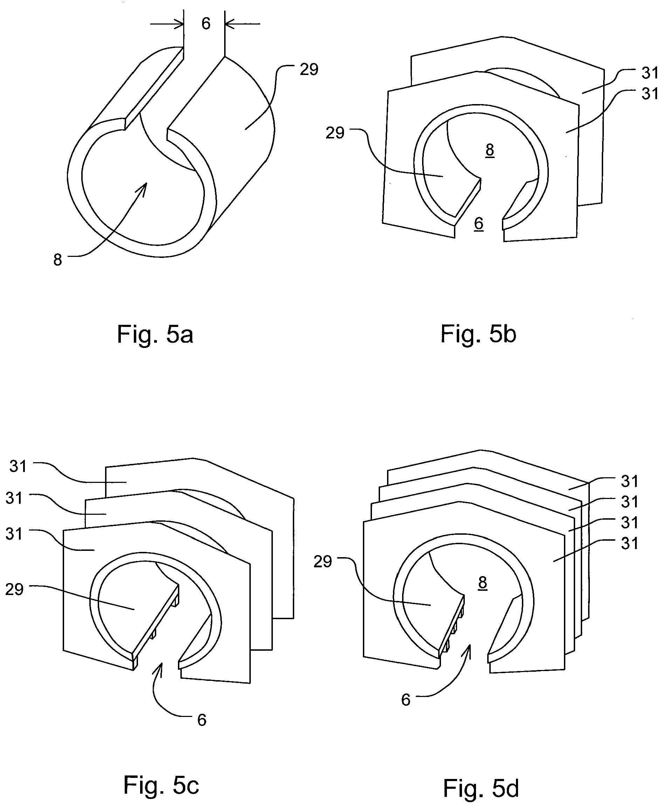

Zur Aufnahme der bei Befüllen des Heizbeutels auftretenden Drücke ist die Wandung oder Begrenzung des Aufnahmeabschnitts in manchen Ausführungsformen der vorliegenden Erfindung entsprechend ausgestaltet. Beispielsweise ist ein Längsspalt des Aufnahmeabschnitts, durch welchen hindurch die Ableitung des Heizbeutels bei dessen Einlegen geführt werden kann (siehe dazu Figur 5a), auf einen Wert aus einem Bereich von 5 mm bis 15 mm Breite, vorzugsweise auf einen Wert aus einem Bereich von 8 mm bis 10 mm, beispielsweise 8 mm begrenzt. Diese Ausgestaltung erlaubt dem Aufnahmeabschnitt, dem Innendruck standzuhalten. Dabei kann das tatsächlich freie Maß der Öffnung des Gehäuses der Heizvorrichtung geringfügig enger oder geringer sein als das Spaltmaß der unten beschriebenen zylindrischen Wandung.

In einigen Ausführungsformen der vorliegenden Erfindung weist die Heizvorrichtung oder der Aufnahmeabschnitt wenigstens ein Verstärkungselement auf, welches angeordnet ist, um eine Verstärkung des Aufnahmeabschnitts gegen radial wirkenden Druck des Heizbeutels zu bewirken. So kann beispielsweise eine zylindrische Wandung des Aufnahmeabschnitts an einem Äußeren des Aufnahmeabschnitts in radialer Richtung mit einem oder mehreren, z.B. 2 oder 3 oder 4 oder mehr, Verstärkungselementen wie beispielsweise Klammern aus Stahl versehen sein.

Ergänzend oder alternativ zu den vorstehend genannten Verstärkungselementen ist die Wandung des Aufnahmeabschnitts in manchen Ausführungsformen der vorliegenden Erfindung zur Verstärkung gegen mechanische Belastung durch den Heizbeutel aus Keramik gefertigt oder weist Keramik auf.

In einigen Ausführungsformen der vorliegenden Erfindung weist die Sensorik der Heizvorrichtung einen Sensor auf, der geeignet und eingesetzt ist zum Erfassen einer Längenausdehnung.

Der Sensor der Sensorik der Heizvorrichtung bestimmt oder meldet in manchen Ausführungsformen der vorliegenden Erfindung die Lage des Heizbeutels relativ zu einem Aufnahmezustand des Heizbeutels vor seiner Längenausdehnung. Beispielsweise bestimmt oder meldet der Sensor eine spätere Ausdehnung des Heizbeutels anhand dessen späterer Lage, Position oder Erstreckung gemessen an seiner früheren Lage, Position oder Erstreckung.

In gewissen Ausführungsformen der vorliegenden Erfindung weist die Sensorik ein verschiebbar vorgesehenes oder gelagertes Bauteil auf, welches beispielsweise als ein mechanischer Tastsensor ausgestaltet ist und im Folgenden vereinfachend aber nicht einschränkend als solches bezeichnet ist. Mittels des mechanischen Tastsensors wird in bestimmten Ausführungsformen der vorliegenden Erfindung eine erkannte Längenerstreckung signalisiert, indem der mechanische Tastsensor, vorzugsweise auf mechanische oder andere Weise, einen Schalter oder eine Vorrichtung wie beispielsweise einen Mikroschalter betätigt. Die Betätigung dieses Schalters oder dieser Vorrichtung führt direkt oder indirekt zu den hierin beschriebenen Folgen des Alarms, wie einen Pumpenstopp, eine Verlangsamung der Pumpe oder andere geeignete Maßnahmen.

In manchen dieser Ausführungsformen weist die Heizvorrichtung ferner eine zusätzliche Überprüfungsvorrichtung auf, welche angeordnet ist, um bei ihrer Betätigung die korrekte Funktion der Sensorik, des verschiebbar gelagerten Bauteils wie beispielsweise dem mechanischen Tastsensor, und/oder des Schalters/der Vorrichtung einzeln oder in beliebigen Kombinationen zu testen. Beispielsweise ist die Überprüfungsvorrichtung in einigen Ausführungsformen der vorliegenden Erfindung als ein mechanisches oder hydraulisches Element ausgestaltet, das angeordnet ist um wie der mechanische Tastsensor, aber unabhängig hiervon, den Schalter, beispielsweise den Mikroschalter, zu betätigen, oder sie weist ein solches Element auf. Mittels Betätigen der Überprüfungsvorrichtung ist es möglich, die Funktion des Schalters zu testen.

In manchen Ausführungsformen der vorliegenden Erfindung ist die Überprüfungsvorrichtung angeordnet, um derart auf den mechanischen Tastsensor einzuwirken oder diesen zu bewegen, dass dieser seinerseits den Schalter betätigt. Bei dieser Anordnung der Überprüfungsvorrichtung ist es bei dessen testweiser Betätigung möglich, sowohl die Funktion oder Bewegbarkeit des mechanischen Tastsensors als auch die Funktion des Schalters zu überprüfen.

Ein Funktionstest für den Schalter - mit oder ohne gleichzeitiger Testung des mechanischen Tastsensors - kann automatisch und beispielsweise bei jeder Inbetriebnahme der Heizvorrichtung ablaufen.

Die Überprüfungsvorrichtung kann einen Hubmagnet und ein Rotationselement aufweisen oder hieraus bestehen. Der Hubmagnet eignet sich vorteilhaft zu seiner kontaktfreien Betätigung auch von einem Äußeren der Heizvorrichtung aus. Das Rotationselement erlaubt es, mittels des Hubmagnets, oder auf andere Weise, auf Platz sparende Weise auf den mechanischen Tastsensor einzuwirken.

In manchen Ausführungsformen der vorliegenden Erfindung überträgt das Rotationselement eine Linearbewegung eines ersten Elements, z. B. einer Stange, eines Hubmagnets oder eines Stößels, auf ein zweites Linearelement. Das Rotationselement und die beiden Linearelemente können in einer Ebene liegen. Das Rotationselement kann auf einer Achse oder Welle drehbar angeordnet sein, beispielsweise auf einem Stift, auf dem das Rotationselement drehbar gelagert ist (z. B. mittels einer Gleitlagerung).

Das zweite Linearelement kann der Tastpin sein. Das Rotationselement kann in den Tastpin eingreifen um diesen bei der Drehung des Rotationselements zu verschieben.

In manchen Ausführungsformen der vorliegenden Erfindung ist das Rotationselement ausgestaltet, um zwar den Tastpin bei Drehung verschieben zu können (indem das Rotationselement beispielsweise an einem Anschlag des Tastpins anliegt), nicht jedoch selbst bewegt zu werden durch ein Verschieben des Tastpins, welches nicht durch das Rotationselement veranlasst ist.

In einigen Ausführungsformen der vorliegenden Erfindung ist die Verformung oder Längenausdehnung des Heizbeutels in axialer Richtung ein Maß für die Werkstoffbeanspruchung des Heizbeutels oder hierauf - insbesondere vollständig oder im Wesentlichen vollständig - zurückzuführen. Eine Überwachung der Längsausdehnung des Heizbeutels kann einen Rückschluss auf die im Moment der Überwachung herrschende Werkstoffbeanspruchung geben. Diese kann mit zuvor gespeicherten Werten verglichen werden. Bei Erreichen oder Überschreiten von zulässigen, meist hinterlegten oder gespeicherten, Grenzwerten für die Werkstoffbeanspruchung kann ein Alarm ausgegeben werden oder der Betrieb einer Vorrichtung, beispielsweise der Pumpe, mittels welcher der Heizbeutel befüllt wird, gestoppt oder verlangsamt werden.

In manchen Ausführungsformen der vorliegenden Erfindung weist die Heizvorrichtung eine Vorrichtung zum Ausgeben eines Alarms oder eine Vorrichtung zum Stoppen oder Verlangsamen einer Pumpe, die die Dialysierflüssigkeit durch den Heizbeutel fördert, oder beides auf. Dabei sind die Vorrichtungen konfiguriert oder eingerichtet oder programmiert, einen Alarm auszugeben oder die Pumpe zu stoppen, wenn die Sensorik eine vorbestimmte Verformung des Heizbeutels, eine vorbestimmte Lage des Heizbeutels im Aufnahmeabschnitt, eine vorbestimmte Werkstoffbeanspruchung oder beliebige Kombinationen hiervon feststellt.

In bestimmten Ausführungsformen der vorliegenden Erfindung ist ein Dialysierflüssigkeits-Schlauchsatz oder -system in die Heizvorrichtung eingelegt. Der Dialysierflüssigkeits-Schlauchsatz weist - vorzugsweise integral mit dem Schlauchsatz hergestellt - wenigstens einen Heizbeutel auf. Der Heizbeutel ist vorgesehen, um hierin mittels der Heizvorrichtung Dialysierflüssigkeit aus dem Dialysierflüssigkeits-Schlauchsatz zu erwärmen.

Das hierin zur Dialysierflüssigkeit Ausgeführte trifft in bestimmten Ausführungsformen der vorliegenden Erfindung auch auf Substituat zu. Ebenso trifft das hier zu Dialysierflüssigkeits-Schlauchsätzen Ausgeführte in bestimmten Ausführungsformen der vorliegenden Erfindung auch auf Substituat-Schlauchsätze zu.

In manchen erfindungsgemäßen Ausführungsformen ist das Dialysierflüssigkeits-Schlauchsystem als Einwegprodukt ausgestaltet.

In einigen erfindungsgemäßen Ausführungsformen ist das Dialysierflüssigkeits-Schlauchsystem ein Dialysierflüssigkeitsschlauch.

In manchen Ausführungsformen der vorliegenden Erfindung weist die medizinische Vorrichtung eine Vorrichtung zum Überwachen einer korrekten Anordnung des Heizbeutels im Aufnahmeabschnitt oder zum Überwachen, dass die Längenausdehnung des Heizbeutels nicht einen vorgegebenen Grenzwert übersteigt, oder beides auf. Die medizinische Vorrichtung weist ferner eine Vorrichtung zum Ausgeben eines Alarms oder eine Vorrichtung zum Stoppen oder Verlangsamen einer Pumpe, die die Dialysierflüssigkeit durch den Heizbeutel fördert, oder beides, auf.

In bestimmten erfindungsgemäßen Ausführungsformen der medizinischen Vorrichtung ist diese als Blutreinigungseinrichtung ausgestaltet, beispielsweise als Dialysiervorrichtung, als Filtrationsvorrichtung, als Diafiltrationsvorrichtung oder als Dialysevorrichtung in jeder anderen, dem Fachmann zur Blutreinigung bekannten Ausgestaltung.

Die medizinische Vorrichtung ist in bestimmten erfindungsgemäßen Ausführungsformen zur Anwendung für die kontinuierliche venöse Hämodiafiltration (CVV-HDF) und/oder zur Anwendung für die Akutdialyse konfiguriert.

In gewissen erfindungsgemäßen Ausführungsformen ist die erfindungsgemäße medizinische Vorrichtung mit einem extrakorporalen Blutkreislauf und/oder einem Schlauchsystem verbunden.

Ein Heizbeutel ist in einigen Ausführungsformen der vorliegenden Erfindung jener Abschnitt eines Dialysierflüssigkeits-Schlauchsatzes, welcher durch Befüllen gebrauchsgemäß dilatiert wird oder unter seiner radialen Expansion gefüllt werden kann.

Ein Heizbeutel ist in bestimmten Ausführungsformen der vorliegenden Erfindung jener Abschnitt eines Dialysierflüssigkeits-Schlauchsatzes, welcher eine dünnere Wandstärke als hieran angrenzende Schlauchabschnitte aufweist.

Ein "durchströmbarer" Heizbeutel ist in manchen Ausführungsformen der vorliegenden Erfindung ein Heizbeutel, welcher einen Zufluss und wenigstens eine hiermit verbundene Zufuhrleitung aufweist. Der Heizbeutel weist ferner einen hiervon getrennten Abfluss mit wenigstens einer hiermit verbundenen Abflussleitung auf. Dabei weisen die Zufuhrleitung oder die Abflussleitung oder beide jeweils einen Durchmesser auf, welcher nicht einem maximalen Durchmesser des Heizbeutels bei dessen Gebrauch entspricht.

In einigen Ausführungsformen der vorliegenden Erfindung ist die Erstreckungsrichtung oder Ausdehnungsrichtung des Heizbeutels die Richtung, in welcher er im Gebrauch durchströmt wird. In manchen Ausführungsformen ist dies die Richtung seiner Längsachse oder Längserstreckung. Wo immer hierin von einer Richtung oder Achse die Rede ist, so kann eine Ausdehnung in einer Richtung entlang der Erstreckung oder Achse gemeint sein.

Unter der Länge des Heizbeutels wird in manchen Ausführungsformen der vorliegenden Erfindung eine Längserstreckung des Heizbeutels verstanden, die dieser im Gebrauch annehmen kann. In einigen Ausführungsformen der vorliegenden Erfindung wird unter der Länge die Längserstreckung des Heizbeutels verstanden, die der Heizbeutel vor seinem Platzen maximal annehmen kann wenn seine radiale Ausdehnung dadurch verhindert oder begrenzt ist, dass der gesamte Heizbeutel radial am Aufnahmeabschnitt anliegt oder anliegen könnte.

Unter der Länge des Aufnahmeabschnitts der Heizvorrichtung wird in bestimmten Ausführungsformen der vorliegenden Erfindung jene Abmessung des Aufnahmeabschnitts in dessen Längsrichtung bei sich - vorzugsweise vollständig oder im Wesentlichen - nicht veränderndem Querschnitt oder innerem Umfang des Aufnahmeabschnitts verstanden, entlang welcher sich der Heizbeutel anlegt oder selbst dann noch anlegen könnte, wenn er sich aufgrund zunehmenden Beutelinnendrucks dehnt.

Ein "Feststellen" oder "Überwachen" wie hierin verwendet kann ein Überprüfen oder Bestimmen sein oder umfassen, ob ein Sensor das Vorliegen eines Abschnitts des Heizbeutels an einer vorbestimmten Stelle des Aufnahmeabschnitts oder der Heizvorrichtung erkennt oder nicht.

Ein "Feststellen" oder "Überwachen" wie hierin verwendet bedeutet in manchen Ausführungsformen der vorliegenden Erfindung nicht das alleinige Feststellen, Überwachen oder Überprüfen, ob ein Heizbeutel in den Aufnahmeabschnitt oder in die Heizvorrichtung eingelegt ist oder nicht. "Alleinig" kann hierbei als ein digitaler Zustand verstanden werden. Beispielsweise wird festgestellt, überwacht oder überprüft, ob ein Heizbeutel überhaupt in den Aufnahmeabschnitt oder in die Heizvorrichtung eingelegt ist ("Ja") oder nicht ("Nein"). In diesen Ausführungsformen bedeutet ein "Feststellen" oder "Überwachen" auch ein Ermitteln, wo der Heizbeutel oder ein Abschnitt hiervon vorliegt.

Ein "Feststellen" oder "Überwachen" wie hierin verwendet bedeutet in gewissen Ausführungsformen der vorliegenden Erfindung das Feststellen, Überwachen oder Überprüfen, ob ein Heizbeutel überhaupt in den Aufnahmeabschnitt oder in die Heizvorrichtung eingelegt ist oder nicht (sein Vorliegen beispielsweise an einem bestimmten Ort oder Position nachweisbar ist), nicht aber, an welchem Ort oder in welcher Lage innerhalb der Heizvorrichtung der Heizbeutel vorliegt.

Das Feststellen oder Überwachen kann messtechnisch erfolgen, die Sensorik entsprechend ausgestaltet sein.

In einigen Ausführungsformen der vorliegenden Erfindung weist die Heizvorrichtung wenigstens eine Fixiereinrichtung zum Fixieren des unteren Schlauchabschnitts an der Heizvorrichtung und/oder zum Sicherstellen auf, dass das untere Ende des Beutels bestimmungsgemäß oder vorbestimmt am unteren Ende der Heizvorrichtung festgelegt ist und/oder dort liegen bleibt.

Beispielsweise kann hierzu die Heizvorrichtung, etwa an ihrer Vorderseite, eine Klemmeinrichtung haben, in welche ein Abschnitt des unteren Schlauchabschnitts, der aus dem Spalt aus der Vorderseite herausgeführt wird, eingeklemmt oder auf andere Weise als durch Klemmwirkung fixiert wird. Es kann vorgesehen sein, hier einen Formschluss und/oder Kraftschluss zwischen dem unteren Schlauchabschnitt und der Heizvorrichtung, beispielsweise deren Vorderseite, zu schaffen.

Hierzu kann in einer bevorzugten Ausführungsform der vertikale Spalt oder der Längsspalt an seinem unteren Ende U-förmig (beispielsweise um eine kurze Distanz) nach oben aufsteigend weitergeführt werden, beispielsweise um ein Mehrfaches des Schlauchdurchmessers des unteren Schlauchabschnitts. Hierdurch kann der untere Schlauchabschnitt nach korrektem Einlegen des Heizbeutels nach oben umgelenkt werden. Die Klemmvorrichtung kann dabei optional vorteilhaft als Zugentlastungselement wirken, mittels der der umgelenkte Schlauchabschnitt an der Heizvorrichtung sicher festgelegt werden kann.

Zusätzlich oder alternativ kann der untere Schlauchabschnitt ein Fixierungselement (beispielsweise eine Kunststoffhülse, beispielsweise mit mindestens einem an oder um seinem Umfang herum verlaufenden Absatz oder Vorsprung) aufweisen. Das Fixierungselement ermöglicht eine formschlüssige Festlegung des unteren Schlauchabschnitts an der Heizvorrichtung (beispielsweise am Spalt, in der Klemmvorrichtung soweit vorgesehen, usw.). Die Klemmvorrichtung kann dabei, falls vorgesehen, als Widerlager wirken und Zugkräfte am Schlauch aufnehmen.

Alternativ oder ergänzend ist in einigen erfindungsgemäßen Ausführungsformen eine kraftschlüssige Fixierung des Schlauchs mittels eines bekannten halboffenen oder längs geschlitzten, rinnenförmigen Schlauchführungselements vorgesehen, in das der untere Schlauchabschnitt unter elastischer Verformung hineingedrückt und damit kraftschlüssig festgelegt werden kann.

Manche oder alle erfindungsgemäßen Ausführungsformen können einen, mehrere oder alle der oben und/oder im Folgenden genannten Vorteile aufweisen.

Ein erfindungsgemäßer Vorteil besteht darin, dass ein Platzen des Heizbeutels aufgrund hohen Innendrucks dadurch verhindert werden kann, dass dieser entlang seiner gesamten Länge - und selbst noch nach Ausdehnung des Heizbeutels - radial oder in Umfangsrichtung vom Aufnahmeabschnitt gestützt wird. Eine Ausdehnung des Heizbeutels ist somit allenfalls in dessen Längsrichtung möglich. Einer lokalen Überbeanspruchung und einer ungleichmäßigen Beanspruchung des Heizbeutels wird hierdurch vorgebeugt.

Ein weiterer mit der vorliegenden Erfindung erzielbarer Vorteil besteht darin, dass mittels der Sensorik eine Kontrolle darüber erfolgen kann, ob der Heizbeutel korrekt eingelegt wurde. Ferner kann mittels der Sensorik überwacht werden, dass sich der Heizbeutel nicht unbemerkt über ein vorgegebenes Maß hinaus ausdehnen oder erstrecken kann. Da ein solches Ausdehnen mittels der Sensorik erkannt werden würde, können erforderliche Maßnahmen rechtzeitig ergriffen werden.

Ein wiederum weiterer Vorteil der vorliegenden Erfindung kann darin bestehen, dass die Funktionstüchtigkeit der Sensorik mittels der Überprüfungsvorrichtung auf einfache und zuverlässige Weise überprüft werden kann. Dies kann getrennt oder gemeinsam für den mechanischen Tastsensor und den Schalter oder eine entsprechende Vorrichtung erfolgen.

Die Überprüfungsvorrichtung kann dabei auf geringem Bauraum vorgesehen sein. Sie kann kontaktlos von außerhalb der Heizvorrichtung betätigt werden.

Bei manchen erfindungsgemäßen Ausführungsformen besteht ein Vorteil darin, dass in der Praxis der Heizbeutel vom Anwender zuverlässig ganz bis nach unten bis zum Ende oder Anschlag in die Heizvorrichtung eingezogen wird. Ferner kann der Heizbeutel in einigen erfindungsgemäßen Ausführungsformen nach korrekt erfolgtem Einlegen im Verlauf des weiteren Aufrüsten des Schlauchsatzes an der medizinischen Vorrichtung nicht versehentlich wieder ein Stück nach oben herausgezogen werden, so dass das untere Ende des Beutels nicht definiert am unteren Ende der Heizvorrichtung festgelegt ist. Auch dies kann mit erfindungsgemäßen Ausführungsformen vorteilhaft vermieden werden.

Die vorliegende Erfindung wird im Folgenden anhand der beigefügten Zeichnung, in welcher identische Bezugszeichen gleiche oder ähnliche Bauteile bezeichnen, exemplarisch erläutert. In der Zeichnung gilt:
- **Fig. 1**: zeigt schematisch in einer Schnittdarstellung eine erfindungsgemäße Heizvorrichtung mit einem Heizbeutel und einer Sensorik zum Detektieren des Heizbeutels;
- **Fig. 2**: zeigt einen Ausschnitt der erfindungsgemäßen Heizvorrichtung aus Fig. 1;
- **Fig. 3a, b**: zeigen schematisch die Sensorik aus Fig. 2 in nicht-aktivierter bzw. aktivierter Testposition;
- **Fig. 4**: zeigt schematisch das Prinzip einer Lichtschranke zum erfindungsgemäßen Detektieren des Heizbeutels;
- **Fig. 5a-d**: zeigen schematisch eine zylindrische Wandung der Heizvorrichtung, teilweise mit Verstärkungselementen;
- **Fig. 6a-g**: zeigen schematisch vereinfacht Varianten zur druckfesten Aufnahme des Heizbeutels in der erfindungsgemäßen Heizvorrichtung; und
- **Fig. 7**: zeigt schematisch eine erfindungsgemäße medizinische Vorrichtung für die Dialyse, mit einem extrakorporalen Blutkreislauf, einer erfindungsgemäßen Heizvorrichtung und einem Dialysierflüssigkeits-Schlauchsatz.

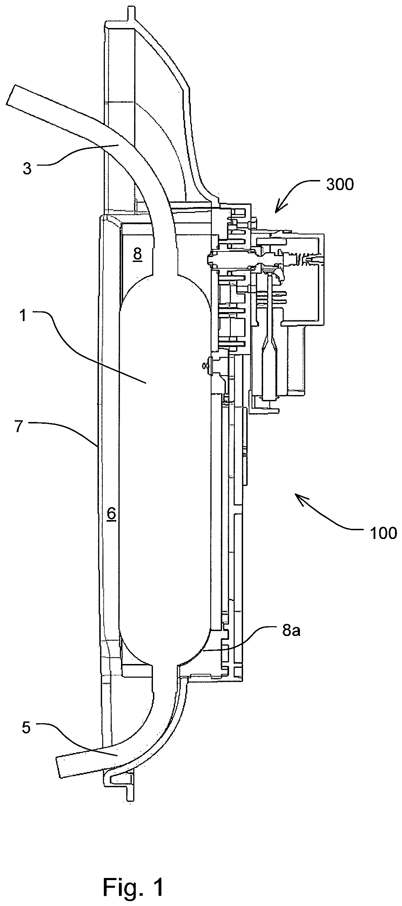

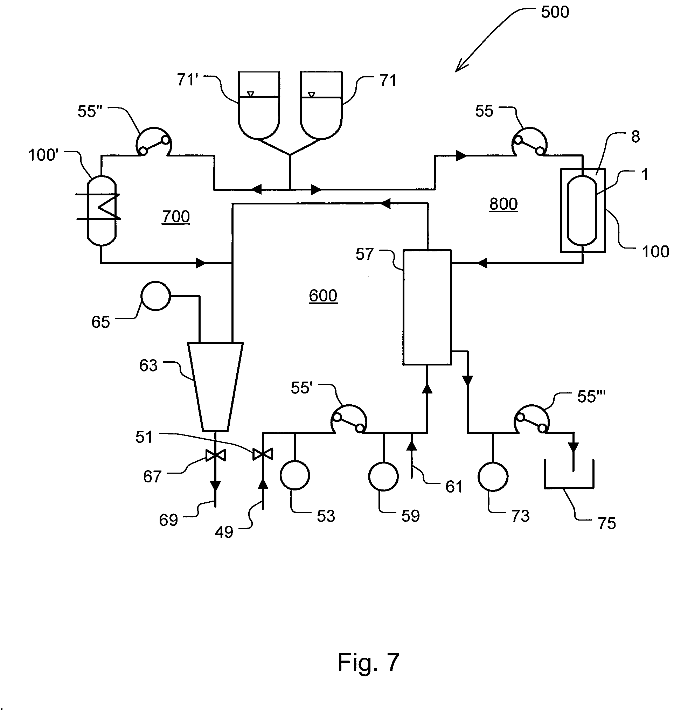

**Fig. 1** zeigt schematisch in einer Schnittdarstellung eine erfindungsgemäße Heizvorrichtung 100 mit einem eingelegten Heizbeutel 1. Der Heizbeutel 1 weist einen oberen Schlauchabschnitt 3 als Zuleitung und einen unteren Schlauchabschnitt 5 als Ableitung (oder umgekehrt) auf, die an einen Dialysierflüssigkeits-Schlauchsatz 800 (siehe Fig. 7) angeschlossen werden können oder damit verbunden sind.

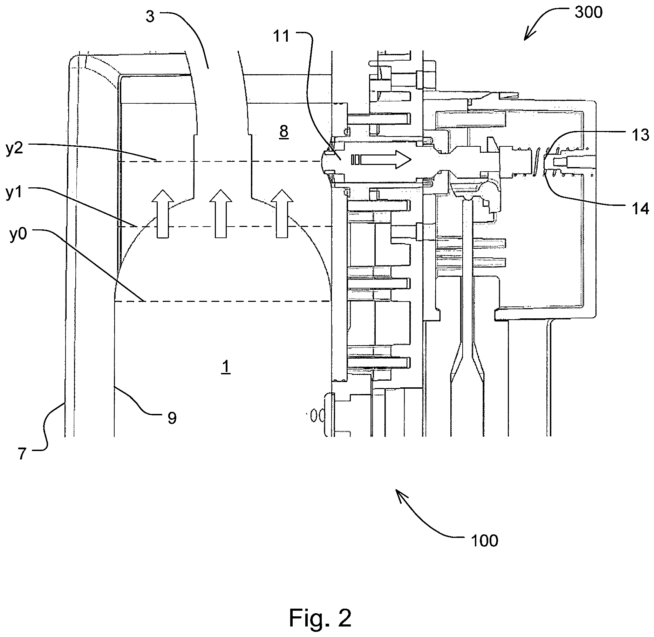

Weiterhin weist die Heizvorrichtung 100 eine Sensorik 300 auf, mit der ein Ausdehnen des Heizbeutels 1, z. B. verursacht durch einen erhöhten Fluid-Innendruck, in Längsrichtung detektiert werden kann (wegen der Sensorik siehe auch Fig. 2).

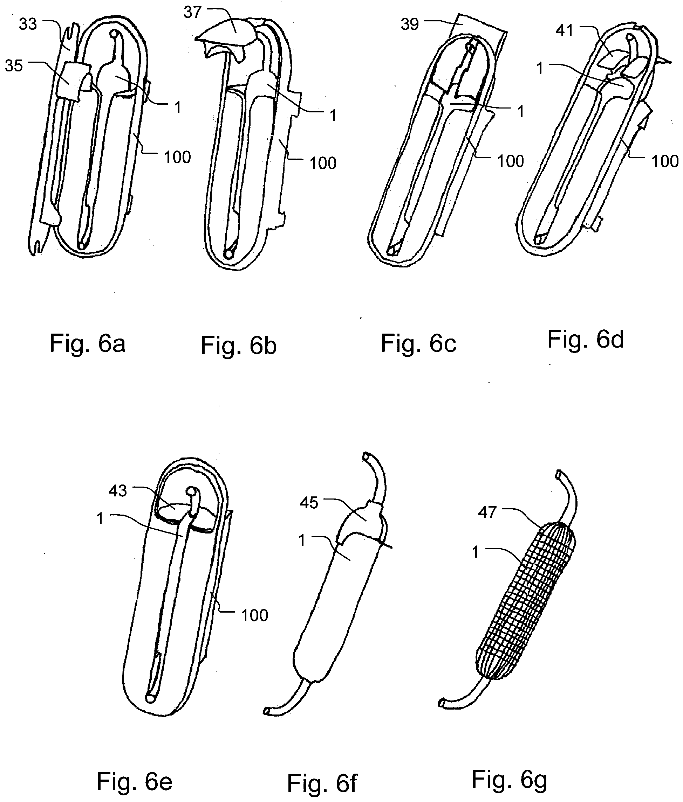

Der Heizbeutel 1 wird in radialer Umfangsrichtung nahezu vollständig von der Heizvorrichtung 100 umschlossen. Lediglich ein Längsspalt 6 verbleibt als Öffnung auf der Vorderseite 7 der Heizvorrichtung 100. Durch den Längsspalt 6 kann der untere Schlauchabschnitt 5 in einen hinter dem Längsspalt 6 gelegenen Aufnahmeabschnitt 8 hindurch geschoben werden, um den Heizbeutel 1 bequem von oben nach unten in die Heizvorrichtung einführen bzw. einlegen zu können. Der Längsspalt 6 ist analog den Fig. 6a bis 6e ausgeführt.

Durchströmt die Dialysierflüssigkeit den Heizbeutel 1, entweder durch den oberen Schlauchabschnitt 3 als Fluideintritt oder Zufuhrleitung und den unteren Schlauchabschnitt 5 als Fluidaustritt oder Abflussleitung, oder umgekehrt, so wird die Dialysierflüssigkeit von der Heizvorrichtung 100 geregelt erwärmt.

In der Darstellung der Fig. 1 kann sich der Heizbeutel 1 nur nach oben (bezogen auf Fig. 1) und damit in seiner Längserstreckung ausdehnen, denn radial oder in Umfangsrichtung ist der Heizbeutel 1 entlang seiner gesamten Länge und darüber hinaus von der Wandung des Aufnahmeabschnitts 8 begrenzt, zudem ist der Heizbeutel 1 mit seinem unteren Stirn- oder Fußende durch einen Bodenabschnitt 8a begrenzt. Der Bodenabschnitt 8a ist in Fig. 1 als gewölbt zu erkennen. Er ist damit der Form des Heizbeutels 1 in dessen gefüllten Zustand nachempfunden. Jede andere Form des Bodenabschnitts 8a ist jedoch ebenfalls von der vorliegenden Erfindung umfasst.

Die vorliegende Erfindung weist in bestimmten Ausführungsformen eine in den Figuren nicht gezeigte Fixiereinrichtung auf zum Sicherstellen, dass das untere Ende des Beutels bestimmungsgemäß am unteren Ende der Heizvorrichtung festgelegt ist und/oder dort liegen bleibt.

Beispielsweise kann hierzu das untere Ende des Heizbeutels 1 in bestimmten erfindungsgemäßen Ausführungsformen am unteren Ende der Heizvorrichtung 100 festgelegt werden, indem der untere Schlauchabschnitt 5, der aus dem Spalt 6 aus der Vorderseite 7 der Heizvorrichtung 100 herausgeführt wird, durch Formschluss und/oder Kraftschluss an der Vorderseite 7 der Heizvorrichtung 100 definiert festgelegt wird. Hierzu kann in einer bevorzugten Ausführungsform der vertikale Spalt 6 an seinem unteren Ende U-förmig (beispielsweise um eine kurze Distanz) nach oben aufsteigend weitergeführt werden, beispielsweise um ein Mehrfaches des Schlauchdurchmessers des unteren Schlauchabschnitts 5. Hierdurch kann der untere Schlauchabschnitt 5 nach korrektem Einlegen des Heizbeutels 1 nach oben umgelenkt werden, wobei eine Klemmvorrichtung an der Vorderseite 7 der Heizvorrichtung 100 als Zugentlastungselement vorgesehen ist, mittels der der umgelenkte Schlauchabschnitt 5 an der Vorderseite 7 der Heizvorrichtung 100 sicher festgelegt werden kann.

Zusätzlich oder alternativ kann der untere Schlauchabschnitt 5 ein in den Figuren nicht gezeigtes Fixierungselement (beispielsweise eine Kunststoffhülse, beispielsweise mit mindestens einem an oder um seinem Umfang herum verlaufenden Absatz oder Vorsprung) aufweisen, das eine formschlüssige Festlegung des unteren Schlauchabschnitts 5 in der Klemmvorrichtung ermöglicht, wobei die Klemmvorrichtung als Widerlager wirkt und Zugkräfte am Schlauch aufnimmt.

Alternativ oder ergänzend ist in einigen erfindungsgemäßen Ausführungsformen eine kraftschlüssige Fixierung mittels eines bekannten halboffenen oder längs geschlitzten, rinnenförmigen Schlauchführungselements vorgesehen, in das der untere Schlauchabschnitt unter elastischer Verformung hineingedrückt und damit kraftschlüssig festgelegt werden kann.

**Fig. 2** zeigt einen Ausschnitt aus Fig. 1 mit einer Sensorik 300 zum Detektieren des Heizbeutels 1 oder dessen Lage innerhalb des Aufnahmeabschnitts 8.

In der Ausgangsposition befindet sich der Heizbeutel 1 im Aufnahmeabschnitt 8 in der Position y0. Es herrscht kein Überdruck im Heizbeutel 1. Die Position y0 liegt dort, wo der Heizbeutel 1 gerade noch Kontakt mit der Innenseite 9 des Aufnahmeabschnitts 8 aufweist. Wenn der Fluiddruck steigt, dehnt sich der Heizbeutel 1 aus. Diese Ausdehnung und ihre Richtung werden in Fig. 2 durch drei parallele Pfeile angedeutet. Ein ansteigender Fluiddruck kann durch einen erhöhten Fluidwiderstand stromab der Heizvorrichtung 100, beispielsweise durch einen ansteigenden Filterwiderstand, verursacht werden, oder durch einen erhöhten Fluiddruck stromauf. Aufgrund des ansteigenden Fluiddrucks dehnt sich der Heizbeutel 1 beispielsweise derart aus, dass er noch bis zur Position y1 Kontakt mit der Innenseite 9 hat. Bei noch weiter ansteigendem Fluiddruck hat der Heizbeutel 1 mit der Innenseite 9 Kontakt bis zur Position y2 oder möglicherweise noch höher.

An der Position y2 weist die Sensorik 300 der erfindungsgemäßen Heizvorrichtung 100 einen Sensor auf, der in diesem Ausführungsbeispiel ein mechanisch verschiebbar angeordnetes Element in Gestalt eines Tastpins 11 und einen Mikroschalter (Tastpin 11 mit Mikroschalter werden auch als mechanischer Tastsensor bezeichnet) ist. Wenn die Ausdehnung des Heizbeutels 1 die Position y2 erreicht und der Heizbeutel 1 noch in der Position y2 Kontakt mit der Innenseite 9 hat, so verschiebt der Heizbeutel 1 den Tastpin 11 in Richtung des horizontalen Pfeils. Daraufhin wird der Mikroschalter (hier nicht näher beschrieben) aktiviert, der beispielsweise ein elektrisches Signal ausgeben kann.

Der Tastpin 11 kann vollständig bis zu einem Anschlag 13 verschoben (bzw. eingedrückt) werden. Der Anschlag kann ein Verkanten des Tastpins 11 verhindern. Es kann ferner eine Feder 14 zum Vorspannen oder Rückstellen des Tastpins 11 vorgesehen sein.

Der Mikroschalter kann ein Signal ausgeben, das wiederum einen Alarm in einer angeschlossenen Rechen- und/oder Steuereinheit auslösen kann um einen Fehlerfall anzuzeigen. Durch den Alarm kann eine geeignete Gegenmaßnahme eingeleitet werden. Beispielsweise kann die den Heizbeutel füllende Pumpe gestoppt oder verlangsamt werden um ein Platzen des Heizbeutels 1 aufgrund anhaltender Pumpleistung zu verhindern.

Der Alarm zeigt ein Erreichen und/oder ein Überschreiten eines vorbestimmten Grenzwertes einer Längenausdehnung oder einer Grenzlage des Heizbeutels 1 an. Der Grenzwert wird in diesem Fall an der Position y2 überschritten. Im hier gezeigten Ausführungsbeispiel ist die Position y2 20 mm von der Position y0, die Position y1 ist 10 mm von der Position y0 entfernt. Der Grenzwert y2 gibt somit eine maximal zulässige Verformung des Heizbeutels 1 an, bevor Maßnahmen ergriffen werden oder jedenfalls ein Alarm ergeht.

Die Sensorik 300 kann weiterhin zur Lagekontrolle des Heizbeutels 1 verwendet werden. Beispielsweise kann ein fehlerhaft eingelegter Heizbeutel detektiert werden, indem bereits bei nicht gefülltem Heizbeutel 1 oder vor Beginn der Dialysebehandlung die Sensorik 300 durch den Heizbeutel 1 aktiviert wird.

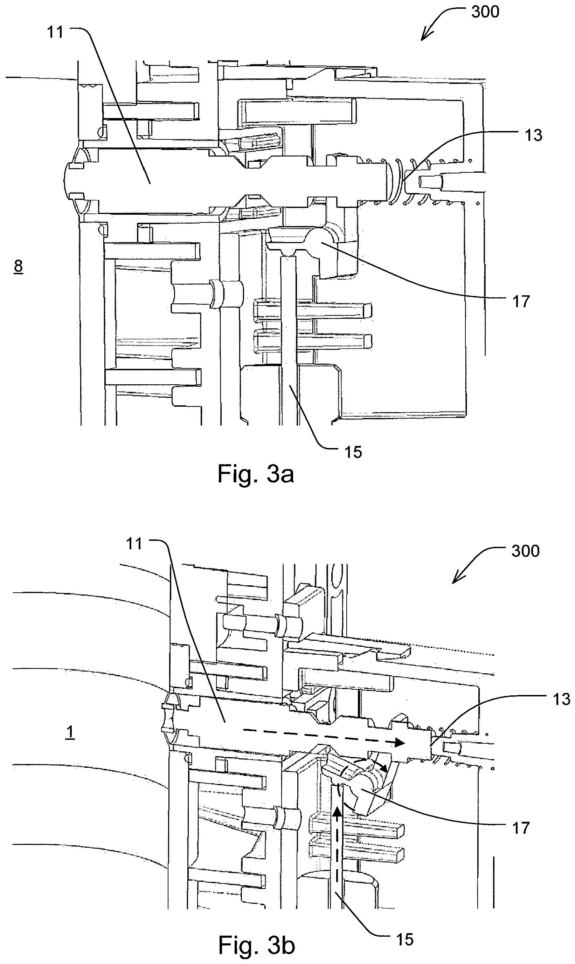

**Fig. 3a, b** zeigen schematisch die Sensorik 300 aus Fig. 2 in nicht-aktivierter (Fig. 3a) bzw. aktivierter (Fig. 3b) Testposition.

Vor Gebrauch der Heizvorrichtung 100 kann die Sensorik 300 auf ihre Funktion hin getestet werden (sogenannter "Selbsttest"). Dies ist insbesondere relevant, sollte der Tastpin 11 mit dem Mikroschalter eine sicherheitsrelevante Schutzeinrichtung eines Gesamtsystems darstellen. Dabei soll möglichst nicht nur der Mikroschalter getestet werden, sondern ebenso die Aktivierung des Mikroschalters durch den Tastpin 11. Eine manuelle Betätigung des Tastpins 11 durch den Anwender wird als nicht sinnvoll betrachtet und kann überdies vergessen werden. Im Folgenden wird ein Mechanismus für einen derartigen Test beschrieben, welcher sich auch automatisch durchführen lässt.

Für den Selbsttest ist eine Überprüfungsvorrichtung vorgesehen. Diese weist im Beispiel der Figuren einen Hubmagnet 15 und ein Rotationselement 17 auf. In anderen als der hier gezeigten Ausführungsform ist es ausreichend, nur einen Magneten, nicht aber auch ein Rotationselement vorzusehen. Der Magnet würde dann den Tastpin 11 auf den Mikroschalter hin ziehen.

In der Darstellung der Fig. 3 hingegen lenkt der Hubmagnet 15, der von außen angesteuert oder aktiviert wird, über ein Rotationselement 17 den Tastpin 11 an. Der Tastpin 11 wird daraufhin verschoben, begrenzt durch den Anschlag 13. Dabei wird der Mikroschalter durch den Tastpin 11 betätigt und löst wie oben beschrieben ein Signal aus.

In Fig. 3b sind mittels von drei Strichlinien die Bewegungen des Tastpins 11, des Hubmagnets 15 und des Rotationselements 17 beim Funktionstest angedeutet. Dabei stehen die linearen Bewegungsrichtungen des Tastpins 11 und des Hubmagnets 1 in einem Winkel zueinander, der hier beispielsweise 90° beträgt. Die Übertragung der Bewegung des Hubmagnets 15 auf den Tastpin 11 erfolgt dabei mittels des Rotationselements 17. Dieser hierdurch mögliche Winkel zwischen den Bewegungen der beiden Elemente 11 und 15, welcher nicht etwa 90° betragen muss sondern auch größer oder kleiner als 90° sein kann, erlaubt eine platzsparende Anordnung der Überprüfungsvorrichtung. Diese kann, z.B. wie hier gezeigt, parallel zur Längserstreckung des Aufnahmeabschnitts 8 und dicht an diesen angelagert angeordnet sein.

Gut zu erkennen ist in den Fig. 3a und 3b, vor allem bei deren Vergleich, dass das Rotationselement 17 bei seiner Drehung den Tastpin 11 mitnimmt, das Rotationselement 17 dabei aber derart ausgestaltet ist, dass der Tastpin 11 unabhängig von einer Drehung des Rotationselements 17 verschoben werden kann.

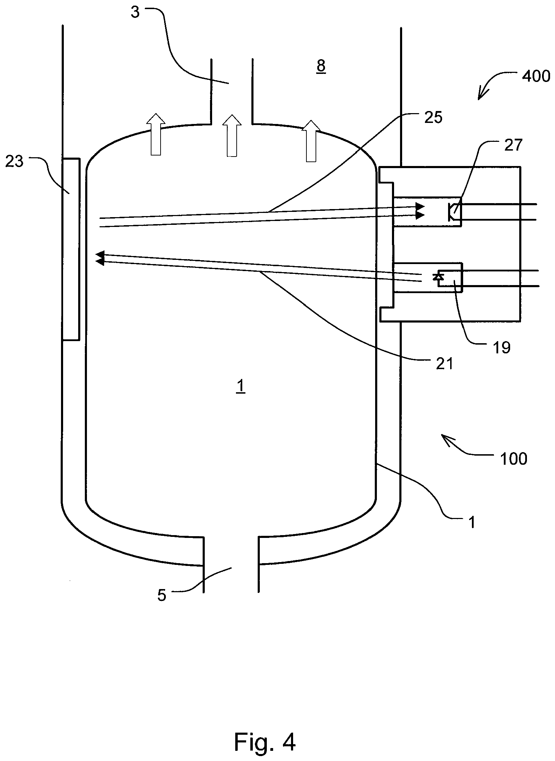

**Fig. 4** zeigt schematisch vereinfacht eine Reflexlichtschranke 400 zum Detektieren des Heizbeutels 1 in einer Heizvorrichtung 100.

Die Reflexlichtschranke 400 kann als Alternative (oder ergänzend als weitere Sicherheitseinrichtung) zu der in den Figuren 1 bis 3 gezeigten Sensorik 300 mit Tastpin 11 und Mikroschalter zum Detektieren des Heizbeutels 1 eingesetzt werden. Die Reflexlichtschranke 400 kann an derselben Position des Aufnahmeabschnitts 8 verwendet werden wie die Sensorik 300.

Mit Hilfe der Reflexlichtschranke 400 lässt sich - wie auch mit der in den Fig. 2 und 3 gezeigten Ausgestaltung - sowohl ein initial falsch eingelegter Heizbeutel 1, wie auch ein sich zu weit ausgedehnter Heizbeutel 1 detektieren.

Weiterhin ist es analog des in Fig. 3 beschriebenen Funktionstests vor Gebrauch der Heizvorrichtung 100 möglich, die Reflexlichtschranke 400 initial vor Gebrauch zu testen. Bei diesem initialen Test wird die Empfindlichkeit der Reflexlichtschranke 400 mit Hilfe einer Schaltung und eines Softwareprogramms (beides wird hier nicht beschrieben oder gezeigt) auf ihren maximalen Wert gestellt. So beträgt die Reichweite der Lichtschranke (maximale Entfernung eines detektierten Gegenstands) beispielsweise mindestens 33 mm, um einen Innenraum eines Zylinders der Heizvorrichtung 100 zu überbrücken. Das von einer Lichtquelle, beispielsweise einer Infrarot-(IR-)Diode 19, ausgesendete Licht 21 wird an der gegenüberliegenden Seite des Zylinders analysiert und bewertet. Alternativ wird es wie in Fig. 4 gezeigt an einem Reflektor 23 reflektiert. Kann das von dem Reflektor 23 reflektierte Licht 25 von der Reflexlichtschranke 400 mit Hilfe einer geeigneten Vorrichtung wie beispielsweise einer Fotodiode 27 (oder Fototransistor) wieder absorbiert werden und ein entsprechendes Spannungssignal von der Fotodiode 27 ausgelesen werden, dann gilt der Funktionstest als bestanden und die Reflexlichtschranke 400 kann zum Detektieren des Heizbeutels 1 in der Heizvorrichtung 100 verwendet werden. Dieser Modus zum Testen, bei dem ein maximaler Wert der Empfindlichkeit oder Reichweite eingestellt ist, wird hierin als Testmodus für die Lichtschranke bezeichnet.

Ist ein Heizbeutel 1 in die Heizvorrichtung 100 eingelegt, so wird die Empfindlichkeit bzw. Reichweite der Reflexlichtschranke 400 mittels des Softwareprogramms reduziert. In diesem Fall sollen das von der IR-Diode 19 ausgesendete Licht 21 weder den gegenüberliegenden Reflektor 23 noch den Schlauchabschnitt 3 oder den Schlauchabschnitt 5 detektieren, sondern ausschließlich die Wand des Heizbeutels 1. Die Reichweite der Reflexlichtschranke 400 könnte dazu beispielsweise auf ca. 5 mm eingestellt werden.

Bei dieser verringerten Reichweite wird der Heizbeutel 1 während des störungsfreien Betriebs, also ohne erhöhten Fluiddruck, nicht detektiert, da er sich unterhalb der Reflexlichtschranke 300 (bezogen auf Fig. 4) befindet. Der Schlauchabschnitt 3 oder der Schlauchabschnitt 5 wird ebenfalls nicht detektiert, da er sich oberhalb ("oberhalb" bezieht sich auf die Figuren und steht allgemein für die Richtung, in welcher sich der Heizbeutel innerhalb der erfindungsgemäßen Heizvorrichtung 100 ausdehnen darf) der verringerten Reichweite befindet, beispielsweise etwa 15 mm von der Reflexlichtschranke 300 entfernt. Die Außenwand des Heizbeutels 1 könnte jedoch bei einem erhöhten Fluiddruck und einem deshalb gedehnten Heizbeutel 1, der sich dann auf der Höhe der Reflexlichtschranke 300 befindet, von der Reflexlichtschranke 300 detektiert werden. Dies gilt sowohl für einen initial fehlerhaft eingelegten Heizbeutel 1 als auch für einen Heizbeutel 1, der sich im Gebrauch auf Grund eines erhöhten Fluiddrucks und/oder einer erhöhten Temperatur des Fluids ausgedehnt hat. Dieser Modus, bei dem eine geringe Empfindlichkeit oder Reichweite zum Detektieren des Heizbeutels 1 eingestellt ist, wird als Betriebsmodus oder Behandlungsmodus der Lichtschranke bezeichnet.

Der Betriebsmodus kann auch verwendet werden, um initial zu erkennen, ob noch ein Heizbeutel 1 aus einer vorherigen Verwendung oder Behandlung eingelegt ist. Diesen Test könnte man direkt nach dem eigentlichen initialen Test (Testmodus) durchführen.

**Fig. 5a****-d** zeigen schematisch eine zylindrische Wandung 29 des Aufnahmeabschnitts 8 der Heizvorrichtung 100 mit unterschiedlich vielen Verstärkungselementen 31.

Die zylindrische Wandung 29 kann ein Rohr mit einem radialen Spalt 6 in Längsrichtung sein, wie in Fig. 5a dargestellt.

Die zylindrische Wandung 29, im Folgenden als Wandung 29 bezeichnet, ist ein integrales Teil der Heizvorrichtung 100.

Die Wandung 29 kann in das Gehäuse der Heizvorrichtung 100, beispielsweise ein Kunststoffgehäuse, integriert sein. Die Wandung 29 umgibt den Aufnahmeabschnitt 8 und auf diese Weise im Wesentlichen den in die Heizvorrichtung 100 eingelegten Heizbeutel 1. Die Wandung 29 verhindert, dass sich der Heizbeutel 1, insbesondere bei Fluidüberdruck im Heizbeutel 1, in Radialrichtung ausdehnt. Je nach Steifigkeit der Wandung 29 erlaubt sie allein oder vorwiegend eine Ausdehnung des Heizbeutels 1 in dessen Längsrichtung (siehe Fig. 1: die Ausdehnung des Heizbeutels 1 erfolgt in Längsrichtung nach oben).

Das Material der Wandung 29 kann Keramik sein oder Keramik aufweisen, z. B. Rubalit. Das Material kann ein Kunststoff sein oder Kunststoff aufweisen (z. B. Polyvinylchlorid, abgekürzt PVC). Es kann ein anderes Material sein.

Die Verstärkungselemente 31, soweit vorhanden, dienen der Verstärkung der Wandung 29, insbesondere zur Erhöhung der Steifigkeit der Wandung 29. Insbesondere bei einer Wandung 29 aus Kunststoff kann das wenigstens eine Verstärkungselement 31 die Steifigkeit deutlich erhöhen.

Die Anordnung und die Anzahl der Verstärkungselemente 31 um die Wandung 29 können unterschiedlich sein. In den Figuren 5b bis 5c werden beispielhafte Anordnungen und Anzahlen von Verstärkungselementen 31 gezeigt, ohne darauf beschränkt zu sein. In Fig. 5b ist die Wandung mit zwei Verstärkungselementen 31, in Fig. 5c mit drei Verstärkungselementen 31 und in Fig. 5d mit vier Verstärkungselementen 31 ausgestaltet. Auch mehr oder weniger Verstärkungselemente als hier gezeigt können erfindungsgemäß vorgesehene sein. Die Verstärkungselemente 31 können aus Metall, beispielsweise ein Stahl, oder aus einem anderen Material gefertigt sein.

Die Verstärkungselemente 31 können zwischen 1 mm und 5 mm dick sein, beispielsweise 2 mm.

Die Verstärkungselemente können Rippenform haben. Sie können insbesondere mit einer Haupterstreckungsrichtung angeordnet sein, welche senkrecht zur Längsrichtung des Aufnahmeabschnitts steht.

Die zylindrische Wandung 29 des Aufnahmeabschnitts 8 der Heizvorrichtung 100 kann als separates Bauteil ausgestaltet sein, sie kann aber auch integral mit der Heizvorrichtung 100 ausgestaltet sein. Die zylindrische Wandung 29 kann beispielsweise als Hülse, insbesondere als nachrüstbare Hülse ausgestaltet sein.

**Fig. 6a****-g** zeigen schematisch vereinfacht verschiedene Varianten zur druckfesten Aufnahme des Heizbeutels 1 in der Heizvorrichtung 100.

In Fig. 6a wird der Heizbeutel 1 mittels einer seitlichen Tür 33 und einer in dieser Tür integrierten Abdeckung 35 für den oberen Teil des Heizbeutels 1 in der Heizvorrichtung 100 fixiert. Bei einem erhöhten Fluiddruck im Heizbeutel 1 wird ein Ausdehnen des Heizbeutels 1 durch diese Fixierung in radialer Richtung des Heizbeutels 1 verhindert oder zumindest beschränkt.

In Fig. 6b wird der Heizbeutel 1 mittels von oben herab geklappter Klappe 37 in der Heizvorrichtung 100 fixiert.

In Fig. 6c wird der Heizbeutel 1 mittels eines in Längsrichtung der Heizvorrichtung 100 verschiebbaren Schiebers 39 in der Heizvorrichtung 100 fixiert.

In Fig. 6d wird der Heizbeutel 1 mittels eines Schwenkschiebers 41 oder Drehschiebers in der Heizvorrichtung 100 fixiert.

In Fig. 6e wird der Heizbeutel 1 mittels einer Abdeckung 43 in der Heizvorrichtung 100 fixiert.

In Fig. 6f wird der Heizbeutel 1 mittels eines auf dem Heizbeutel 1 angebrachten Clips 45 in der Heizvorrichtung 100 (hier nicht dargestellt) fixiert.

In Fig. 6g wird der Heizbeutel 1 mittels eines um den Heizbeutel 1 herum gelegten Netzes 47 in der Heizvorrichtung 100 (hier nicht dargestellt) fixiert.

**Fig. 7** zeigt schematisch eine erfindungsgemäße medizinische Vorrichtung 500 (auch als Behandlungssystem bezeichnet) für die Dialyse, mit einem extrakorporalen Blutkreislauf 600, einem Substituat-Schlauchsatz 700 und einem Dialysierflüssigkeits-Schlauchsatz 800.

Dem Patienten wird mittels eines arteriellen Anschlusses 49 einer arteriellen Leitung des extrakorporalen Blutkreislaufs 600 Blut entnommen. Stromab des arteriellen Anschlusses 49 ist optional ein Absperrhahn 51 angeordnet. Wiederum stromab hiervon wird optional der arterielle Druck mittels eines Drucksensors 53 gemessen, noch weiter stromab ist eine Blutpumpe 55' angeordnet. Zwischen der Blutpumpe 55' und dem Anschluss der arteriellen Leitung an den Dialysator 57 wird optional der Hämofiltrationsdruck mittels eines Drucksensors 59 gemessen. Stromab dieses Drucksensors 59 wird dem Blut optional an einer Zugabestelle 61 Heparin zur Gerinnungshemmung zugegeben.

Im Dialysator 57 findet der Stoffaustausch mit der Dialysierflüssigkeit des Dialysierflüssigkeits-Schlauchsatzes 800, welches den Dialysator 57 als Dialysat verlässt, statt. Dies wird weiter unten näher beschrieben.

Stromab des Dialysators 57 fließt das Blut in eine optionale venöse Tropfkammer 63, in der mittels eines optionalen Drucksensors 65 der venöse Druck gemessen wird. Stromab hiervon ist optional ein Absperrhahn 67 angeordnet. Das Blut wird mittels eines venösen Anschlusses 69 in das Gefäßsystem des Patienten zurückgeführt.

Der Substituat-Schlauchsatz 700 dient dazu, dem Patienten einen Teil des bei der Behandlung entzogenen Flüssigkeitsvolumens, welches dem Blut durch die Filtration im Dialysator 57 entzogen wurde, zu substituieren. Hierzu wird Substituat-Flüssigkeit aus einem Beutel 71 oder 71' entnommen, aus welchem auch die Dialysierflüssigkeit stammen kann. Das Substituat wird im Substituat-Schlauchsatz 700 mittels einer Substituatpumpe 55" in eine Heizvorrichtung 100' gefördert und dort erwärmt, und anschließend dem Blutkreislauf 600 zugeführt.

Der Dialysierflüssigkeits-Schlauchsatz 800, der als Einweg-Schlauchsystem ("Disposable") ausgeführt sein kann, wird von einer Dialysierflüssigkeit aus einem Beutel 71 befüllt. Die Dialysierflüssigkeit wird mittels einer hier nur exemplarisch als Rollenpumpe ausgestalteten Pumpe 55 im Dialysierflüssigkeits-Schlauchsatz 800 gefördert. Dabei kann die Dialysierflüssigkeit aus dem Beutel 71 mittels Schwerkraft und/oder durch Ansaugen mittels der Pumpe 55 zwischen dem Beutel 71 und der stromab der Pumpe 55, also auf der Druckseite der Pumpe 55, gelegenen Heizvorrichtung 1 fließen. Im Heizbeutel 1 wird die Dialysierflüssigkeit mittels der erfindungsgemäßen Heizvorrichtung 100 erwärmt.

Aufgrund des durch die Pumpe 55 aufgebauten Fluiddrucks kann im Schlauchsystem stromab der Pumpe 55 ein Druck von ca. 50 mbar während einer Behandlung eines Patienten entstehen. Dieser Druck kann während einer Behandlung auf 1050 mbar ansteigen. Ist die Strömung durch das nachgeschaltete Schlauchsystem blockiert, beispielsweise durch einen entsprechenden Zustand des Filters, so kann ein maximaler Druck von 3200 mbar erreicht werden.

Weiter stromab der Pumpe 55 und stromab der Heizvorrichtung 1 ist der Dialysator 57 angeordnet. Stromab des Dialysators 57 wird der Filtratdruck optional mittels eines Drucksensors 73 gemessen, weiter stromab wird das Dialysat gemeinsam mit dem Filtrat mittels einer Pumpe 55"' in einen Auffangbehälter 75 gefördert oder verworfen.

**Bezugszeichenliste**

| **Bezugszeichen** | **Beschreibung** |
|---|---|
| 100, 100' | Heizvorrichtung |
| 200 | Schlauchset |
| 300 | Sensorik |
| 400 | Reflexlichtschranke |
| 500 | medizinische Vorrichtung |
| 600 | extrakorporaler Blutkreislauf |
| 700 | Substituat-Schlauchsatz |
| 800 | Dialysierflüssigkeits-Schlauchsatz |
| y0 | Ausgangsposition des Heizbeutels 1 |
| y1 | Zwischenposition des Heizbeutels 1 |
| y2 | Endposition des Heizbeutels 1 mit Aktivierung des Tastpins 11 |
| 1 | Heizbeutel |
| 3 | oberer Schlauchabschnitt |
| 5 | unterer Schlauchabschnitt |
| 6 | Spalt, Längsspalt |
| 7 | Vorderseite der Heizvorrichtung 100 |
| 8 | Aufnahmeabschnitt |
| 8a | Bodenabschnitt |
| 9 | Innenseite des Aufnahmeabschnitts 8 |
| 11 | Tastpin |
| 13 | Anschlag des Tastpins 11 |
| 14 | Feder |
| 15 | Hubmagnet |
| 17 | Rotationselement |
| 19 | IR-Diode |
| 21 | von der IR-Diode ausgesendetes Licht |
| 23 | Reflektor |
| 25 | vom Reflektor reflektiertes Licht |
| 27 | Fotodiode, Fototransistor |
| 29 | zylindrische Wandung |
| 31 | Verstärkungselement |
| 33 | seitliche Tür |
| 35 | integrierte Abdeckung |
| 37 | Klappe |
| 39 | Schieber |
| 41 | Schwenkschieber |
| 43 | Abdeckung |
| 45 | Clip |
| 47 | Netz |
| 49 | arterieller Anschluss |
| 51 | Absperrhahn, arteriell |
| 53 | Drucksensor für arteriellen Druck |
| 55 | Dialysierflüssigkeitspumpe |
| 55' | Blutpumpe |
| 55" | Substituatpumpe |
| 55''' | Dialysatpumpe |
| 57 | Dialysator |
| 59 | Drucksensor für Hämofiltrationsdruck |
| 61 | Zugabestelle für Heparin |
| 63 | venöse Tropfkammer |
| 65 | Drucksensor für venösen Druck |
| 67 | Absperrhahn, venös |
| 69 | venöser Anschluss |
| 71,71' | Beutel |
| 73 | Drucksensor für Filtratdruck |
| 75 | Auffangbehälter |
| | |
| | |
| | |
| | |

## Patentansprüche

1. Heizvorrichtung (100) zum Erwärmen einer Dialysierflüssigkeit oder eines Substituats, welche einen Aufnahmeabschnitt (8) zum Aufnehmen eines von der Dialysierflüssigkeit oder des Substituats zu ihrem/seinem Erwärmen durchströmbaren Heizbeutels (1) aufweist, wobei der Aufnahmeabschnitt (8) ausgestaltet ist, um eine Verformung des Heizbeutels (1) infolge eines Heizbeutelinnendrucks in nur eine Erstreckungsrichtung des Heizbeutels (1) zuzulassen,
**dadurch gekennzeichnet, dass**
die Heizvorrichtung (100) eine Sensorik (300) aufweist zum Feststellen oder Überwachen einer Verformung des Heizbeutels (1) oder der Lage des Heizbeutels (1) in der Heizvorrichtung (100) oder im Aufnahmeabschnitt (8) der Heizvorrichtung (100).

2. Heizvorrichtung (100) nach Anspruch 1, wobei die Heizvorrichtung (100) oder der Aufnahmeabschnitt (8) wenigstens ein Verstärkungselement (31) aufweist, welches angeordnet ist, um eine Verstärkung des Aufnahmeabschnitts gegen radial wirkenden Druck des Heizbeutels zu bewirken.

3. Heizvorrichtung (100) nach einem der vorangegangenen Ansprüche, wobei die Heizvorrichtung (100) ausgestaltet ist, den durchströmbaren Heizbeutel (1) bezogen auf die Umfangsrichtung des Heizbeutels (1) entlang der gesamten Länge des Heizbeutels (1) zu umschließen.

4. Heizvorrichtung (100) nach einem der vorangegangenen Ansprüche, wobei die Heizvorrichtung (100) den durchströmbaren Heizbeutel (1) zylindrisch umschließt.

5. Heizvorrichtung (100) nach einem der vorangegangenen Ansprüche, wobei die Sensorik (300) zur Überwachung der Verformung des Heizbeutels (1) wenigstens einen mechanischen (11) oder einen optischen (19, 23, 25) Sensor aufweist oder hieraus besteht.

6. Heizvorrichtung (100) nach einem der vorangegangenen Ansprüche, mit einer Vorrichtung zum Ausgeben eines Alarms und/oder zum Stoppen einer Pumpe (55, 55"), die die Dialysierflüssigkeit oder das Substituat durch den Heizbeutel (1) fördert, wobei die Vorrichtung konfiguriert ist, einen Alarm auszugeben oder die Pumpe (55, 55") zu stoppen, wenn die Sensorik (300) eine vorbestimmte Verformung des Heizbeutels (1) oder eine vorbestimmte Lage oder Position (y₂) des Heizbeutels (1) im Aufnahmeabschnitt (8) feststellt.

7. Heizvorrichtung (100) nach einem der vorangegangenen Ansprüche, welche ferner eine zusätzliche Überprüfungsvorrichtung (15, 17) aufweist, welche angeordnet ist, um bei ihrer Betätigung einzuwirken auf wenigstens eine Komponente der Sensorik (300), um die Funktion der Sensorik (300) zu testen.

8. Heizvorrichtung (100) nach einem der vorangegangenen Ansprüche, in welche ein Dialysierflüssigkeits-Schlauchsatz (800) oder ein Substituat-Schlauchsatz (700) eingelegt ist, aufweisend wenigstens einen Heizbeutel (1), welcher vorgesehen ist um hierin mittels der Heizvorrichtung (100) Dialysierflüssigkeit aus dem Dialysierflüssigkeits-Schlauchsatz (800) oder Substituat aus dem Substituat-Schlauchsatz (700) zu erwärmen.

9. Set mit
- einer Heizvorrichtung (100) nach einem der Ansprüche 1 bis 8, welche einen Aufnahmeabschnitt (8) für einen Heizbeutel (1) aufweist; und
- wenigstens einem Dialysierflüssigkeits-Schlauchsatz (800) oder einem Substituat-Schlauchsatz (700), der jeweils vorgesehen oder ausgestaltet ist, um in eine Heizvorrichtung (100) nach einem der vorangegangenen Ansprüche eingelegt zu werden, welcher einen durchströmbaren Heizbeutel (1) aufweist;
wobei der Dialysierflüssigkeits-Schlauchsatz (800) oder der Substituat-Schlauchsatz (700) derart ausgewählt ist, dass die Länge des Heizbeutels (1) kleiner ist als die Länge des Aufnahmeabschnitts (8) der Heizvorrichtung (100).

10. Medizinische Vorrichtung (500), aufweisend wenigstens eine Heizvorrichtung (100) nach einem der Ansprüche 1 bis 8, oder hiermit verbunden.

11. Medizinische Vorrichtung (500) nach Anspruch 10, mit einer Vorrichtung zum Überwachen einer korrekten oder vorbestimmten Anordnung des Heizbeutels (1) im Aufnahmeabschnitt (8) oder zum Überwachen einer Längenausdehnung des Heizbeutels (1) nicht über einen vorgegebenen Grenzwert hinaus, mit einer Vorrichtung zum Ausgeben eines Alarms und/oder zum Stoppen oder Verlangsamen einer Pumpe (55, 55"), die die Dialysierflüssigkeit oder das Substituat durch den Heizbeutel (1) fördert.

12. Medizinische Vorrichtung (500) nach Anspruch 10 oder 11, ausgestaltet als Blutbehandlungsvorrichtung, Blutreinigungsvorrichtung oder Dialysevorrichtung.

## Claims

1. A heating apparatus (100) for heating a dialysis liquid or a substitute, which comprises a receiving section (8) for receiving a heating bag (1) through which dialysis liquid or substitute can flow for being heated, wherein the receiving section (8) is embodied for allowing a deformation of the heating bag (1) due to an interior pressure of the heating bag in only one extension direction of the heating bag (1)
**characterized in that**
the heating apparatus (100) comprises a sensor system (300) for determining or monitoring a deformation of the heating bag (1) or the location of the heating bag (1) in the heating apparatus (100) or in the receiving section (8) of the heating apparatus (100).

2. The heating apparatus (100) according to claim 1, wherein
the heating apparatus (100) or the receiving section (8) comprises at least one reinforcing element (31) which is arranged for effecting a reinforcement of the receiving section against a radially acting pressure of the heating bag.

3. The heating apparatus (100) according to any one of the preceding claims, wherein the heating apparatus (100) is embodied for enclosing the flow-through heating bag (1) with respect to the circumferential direction of the heating bag (1) along the whole length of the heating bag (1).

4. The heating apparatus (100) according to any one of the preceding claims, wherein the heating apparatus (100) encloses cylindrically the flow-through heating bag (1).

5. The heating apparatus (100) according to any one of the preceding claims, wherein the sensor system (300) for monitoring the deformation the heating bag (1) comprises or consists of at least one mechanical (11) or one optical (19, 23, 25) sensor.

6. The heating apparatus (100) according to any one of the preceding claims, with an apparatus for outputting an alarm and/or for stopping a pump (55, 55') which conveys the dialysis liquid or the substitute through the heating bag (1), wherein the apparatus is configured for outputting an alarm or for stopping the pump (55, 55') if or when the sensor system (300) determines a predetermined deformation of the heating bag (1) or a predetermined position (y2) of the heating bag (1) in the receiving section (8).

7. The heating apparatus (100) according to any one of the preceding claims, which further comprises an additional checking apparatus (15, 17) which is arranged for, when actuated, acting on at least one component of the sensor system (300) in order to check the function of the sensor system (300).

8. The heating apparatus (100) according to any one of the preceding claims, in which a dialysis liquid tube set (800) or a substitute tube set (700) is inserted, the heating apparatus (100) comprising at least one heating bag (1), which is intended for heating dialysis liquid from the dialysis liquid tube set (800) or substitute from the substitute tube set (700) herein by means of the heating apparatus (100).

9. A set having:
- a heating apparatus (100) according to any one of the claims 1 to 8, which comprises a receiving section (8) for a heating bag (1); and
- at least one dialysis liquid tube set (800) or a substitute tube set (700), which are each intended and embodied for being inserted into a heating apparatus (100) according to any one of the preceding claims and comprising a flow-through heating bag (1);
wherein the dialysis liquid tube set (800) or the substitute tube set (700) is selected such that the length of the heating bag (1) is smaller than the length of the receiving section (8) of the heating apparatus (100).

10. A medical apparatus (500), comprising at least one heating apparatus (100) according to any one of the claims 1 to 8, or connected herewith.

11. The medical apparatus (500) according to claim 10, with an apparatus for monitoring a correct or pre-determined arrangement of the heating bag (1) in the receiving section (8) or for monitoring that a longitudinal expansion of the heating bag (1) does not exceed a predefined limit value, with an apparatus for outputting an alarm and/or for stopping or slowing down a pump (55, 55') which conveys the dialysis liquid or the substitute through the heating bag (1).

12. The medical apparatus (500) according to claim 10 or 11, embodied as a blood treatment apparatus, blood purification apparatus or dialysis apparatus.

## Revendications

1. Dispositif de chauffage (100) destiné à chauffer un liquide de dialyse ou un liquide de substitution, présentant une section de réception (8) destinée à recevoir une poche chauffante (1) susceptible d'être traversée par le liquide de dialyse ou le liquide de substitution, de façon à chauffer le liquide, où la section de réception (8) est conçue de façon à permettre une déformation de la poche chauffante (1) provoquée par une pression interne de la poche chauffante dans une unique direction d'extension de la poche chauffante (1),
**caractérisé en ce que**
le dispositif de chauffage (100) présente une technique sensorielle (300) destinée à constater ou à surveiller une déformation de la poche chauffante (1) ou de la position de la poche chauffante (1) dans le dispositif de chauffage (100) ou dans la section de réception (8) du dispositif de chauffage (100).

2. Dispositif de chauffage (100) selon la première revendication, où le dispositif de chauffage (100) ou la section de réception (8) comprend au moins un élément de renfort (31) disposé de façon à obtenir un renforcement de la section de réception contre la pression de la poche chauffante s'exerçant radialement.

3. Dispositif de chauffage (100) selon l'une quelconque des revendications précédentes, où le dispositif de chauffage (100) est conçu de façon à entourer la poche chauffante (1) susceptible d'être traversée relativement au sens de la circonférence de la poche chauffante (1) tout le long de la poche chauffante (1).

4. Dispositif de chauffage (100) selon l'une quelconque des revendications précédentes, où le dispositif de chauffage (100) entoure la poche chauffante (1) susceptible d'être traversée de façon cylindrique.

5. Dispositif de chauffage (100) selon l'une quelconque des revendications précédentes, où la technique sensorielle (300) destinée à surveiller la déformation de la poche chauffante (1) présente ou est constituée par au moins un capteur mécanique (11) ou un capteur optique (19, 23, 25).

6. Dispositif de chauffage (100) selon l'une quelconque des revendications précédentes, comprenant un dispositif destiné à émettre une alerte et/ou à arrêter une pompe (55, 55'') permettant le transport du liquide de dialyse ou du liquide de substitution au travers de la poche chauffante (1), où le dispositif est configuré de façon à émettre une alarme ou à arrêter la pompe (55, 55'') lorsque la technique sensorielle (300) constate une déformation prédéterminée de la poche chauffante (1) ou un agencement, ou une position (Y2), prédéterminé(e) de la poche chauffante (1) dans la section de réception (8).

7. Dispositif de chauffage (100) selon l'une quelconque des revendications précédentes, qui présente par ailleurs un dispositif de contrôle supplémentaire (15, 17) disposé de façon à agir, lors son actionnement, sur au moins un composant de la technique sensorielle (300) afin de tester la fonction de la technique sensorielle (300).

8. Dispositif de chauffage (100) selon l'une quelconque des revendications précédentes, dans lequel un ensemble de tubes pour liquide de dialyse (800) ou un ensemble de tubes pour liquide de substitution (700) est inséré et qui présente au moins une poche chauffante (1) prévue pour chauffer le liquide de dialyse de l'ensemble de tubes pour liquide de dialyse (800) ou le liquide de substitution de l'ensemble de tubes pour liquide de substitution (700) au moyen du dispositif de chauffage (100).

9. Set comprenant :
- un dispositif de chauffage (100) selon l'une quelconque des revendications 1 à 8, présentant une section de réception (8) pour une poche chauffante (1) ; et
- au moins un ensemble de tubes pour liquide de dialyse (800) ou un ensemble de tubes pour liquide de substitution (700), chacun étant prévu ou conçu de façon à être inséré dans un dispositif de chauffage (100), selon l'une quelconque des revendications précédentes, présentant une poche chauffante (1) susceptible d'être traversée par le liquide ;
où l'ensemble de tubes pour liquide de dialyse (800) ou l'ensemble de tubes pour liquide de substitution (700) est choisi de telle sorte que la longueur de la poche chauffante (1) est inférieure à la longueur de la section de réception (8) du dispositif de chauffage (100).

10. Dispositif médical (500), présentant au moins un dispositif de chauffage (100) selon l'une quelconque des revendications 1 à 8, ou étant connecté à un tel dispositif.

11. Dispositif médical (500) selon la revendication 10, comprenant un dispositif destiné à surveiller un agencement correct ou un agencement prédéfini de la poche chauffante (1) dans la section de réception (8) ou destiné à surveiller qu'une expansion en longueur de la poche chauffante (1) ne dépasse pas une valeur de seuil prédéterminée, comprenant un dispositif destiné à émettre une alarme et / ou à arrêter ou ralentir une pompe (55, 55'') permettant le transport du liquide de dialyse ou du liquide de substitution au travers de la poche chauffante (1).

12. Dispositif médical (500) selon la revendication 10 ou 11, conçu comme appareil de traitement du sang, appareil d'épuration du sang ou appareil de dialyse.
